# FASCICULE DE BREVET EUROPEEN

(11) **EP 0 770 402 B1**
(45) Date de publication et mention de la délivrance du brevet: **03.07.2002**
(21) Numéro de dépôt: 96116642.8
(22) Date de dépôt: 17.10.1996
(51) Int. Cl.: A61M 1/02, A61M 1/36, A61J 1/00

(54) **Poche souple de filtration pour sang**
Blutbeutel mit Filtermitteln
Blood bag with filtering means

(30) Priorité: 26.10.1995 CH 305295
(43) Date de publication de la demande: 02.05.1997
(73) Titulaire: MEDTRONIC ELECTROMEDICS INC, Parker, Colorado 80134 (US)
(72) Inventeur: Rochat, Jean-Denis, 1272 Genolier (CH)
(74) Mandataire: Micheli & Cie

(56) Documents cités:
- EP-A- 0 206 638
- EP-A- 0 484 751
- EP-A- 0 525 492
- EP-A- 0 645 151

## Description

La présente invention concerne une poche souple de filtration jetable, notamment pour équiper un appareil de récupération et de filtration stérile du sang per-et post-opératoire, par exemple du type de celui faisant l'objet du brevet EP 0.525.493 de la même titulaire.

Or il s'est avéré qu'en pratique la poche souple de filtration jetable qui est décrite dans le document précité présente des inconvénients liés à sa structure et à sa réalisation. En effet, il convient de rappeler que ladite poche (par exemple en référence à la figure 1 du brevet EP 0.595.493) comporte deux chambres, sensiblement du même volume, et séparées l'une de l'autre par une paroi interne souple de filtration, par exemple en polyester. Or, cette disposition forme une première chambre de rétention du sang à filtrer qui présente un grand volume entre l'entrée de celui-ci dans ladite chambre et le filtre, et une grande quantité de sang à filtrer s'accumule rapidement dans cette première chambre entre la paroi externe de celle-ci et la paroi interne de filtration, surtout lorsque celle-ci se bouche à cause de la coagulation du sang récupéré pouvant contenir également des particules solides. Ce relativement grand volume de rétention s'avère en pratique désavantageux, d'une part à cause du fait que la surface du filtre en contact avec le sang à filtrer est proportionnellement importante, ce qui diminue la pression différentielle entre les deux chambres qui est destinée à forcer le sang à traverser le filtre, et d'autre part étant donné que de volume de rétention n'est pas récupérable puisqu'il n'est pas accessible depuis le bas de la poche par le siphon situé en aval du filtre.

Ainsi, le but de la présente invention consiste à fournir une poche souple de filtration jetable, notamment pour appareil de récupération et de filtration du sang, qui ne présente pas les inconvénients précités des poches connues.

Le but précité est atteint par la poche souple de filtration, objet de l'invention, et telle que définie dans la revendication 1.

Cette invention sera maintenant décrite plus en détails en référence au dessin annexé illustrant schématiquement et à titre d'exemple une forme d'exécution de la poche de filtration.

La figure 1 en est une vue générale en perspective, partiellement éclatée.

La figure 2 est une vue en plan d'une des parois constitutive de la poche selon la figure 1.

La figure 3 est un schéma en coupe illustrant le fonctionnement de la poche selon la figure 1.

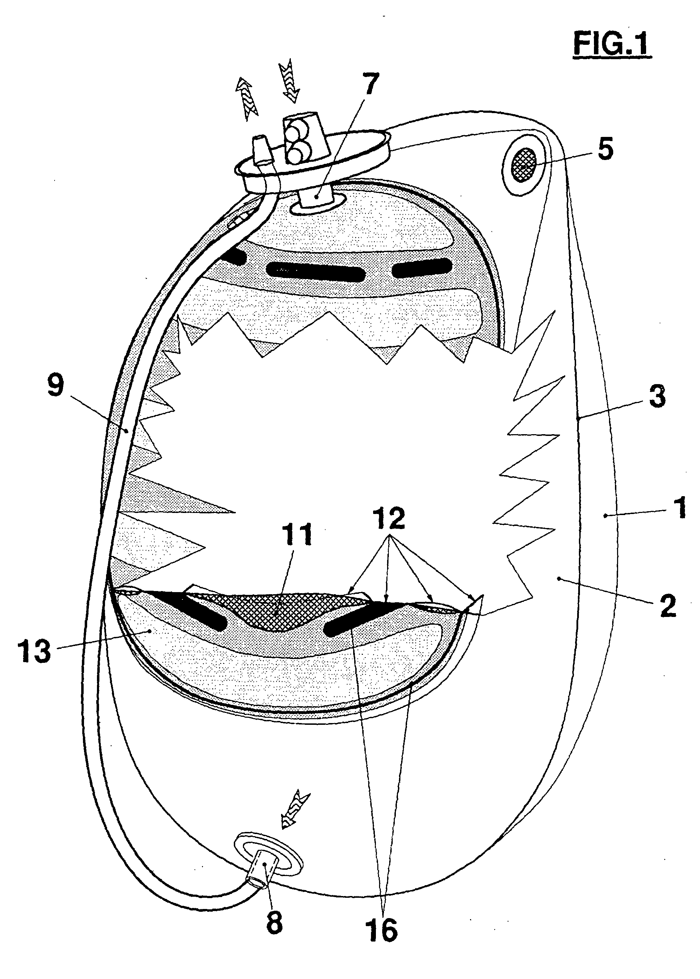

La forme d'exécution de la poche souple filtrante selon l'invention, telle qu'illustrée sur les figures 1 à 3, est formée de manière connue en soi par deux parois 1,2, de préférence en polyvinyl chloride (PVC), assemblées à plat par leurs rebords périphériques 3.

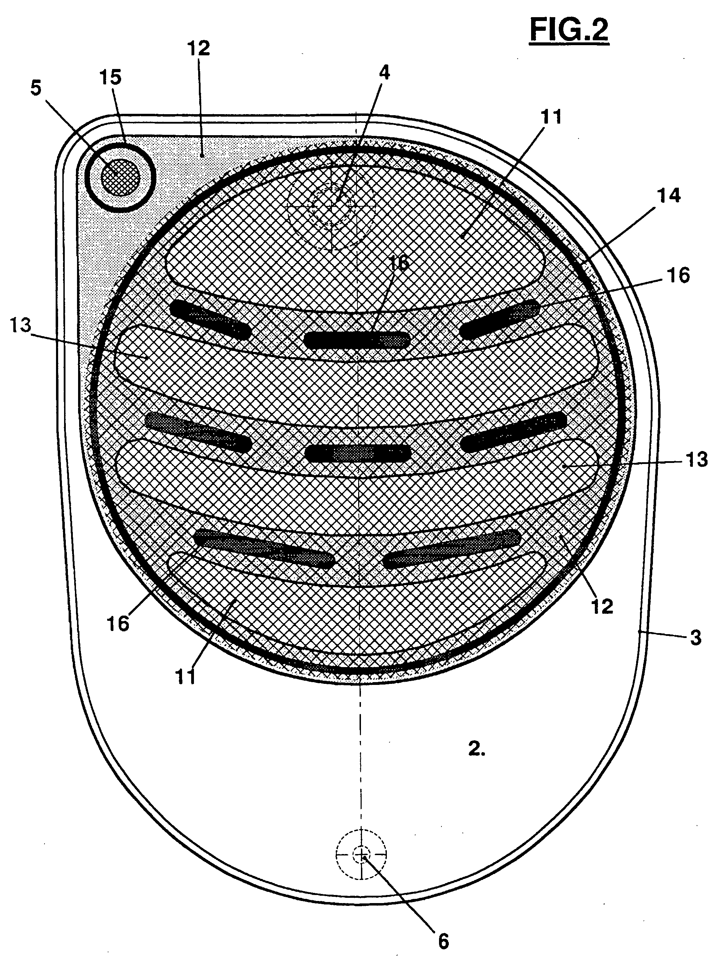

L'une des parois 1 en PVC est simple et ne comporte aucune ouverture ni élément additionnel. Par contre, l'autre paroi 2 en PVC, telle que représentée plus particulièrement sur la figure 2, présente trois ouvertures, une première ouverture 4 pour l'entrée du sang brut à traiter et de l'air d'aspiration, une seconde ouverture 5, située comme la première dans la partie supérieure de la paroi 2, destinée à la sortie de l'air, et une troisième ouverture 6, située dans la partie inférieure de la paroi 2 et servant à la sortie du sang filtré. Sur chacune des ouvertures d'entrée 4, respectivement de sortie 6, du sang est fixé par soudage à la paroi PVC un tube de connexion 7,8 , et la sortie de l'air 5 est munie d'une grille en polypropylène à effet électrostatique et servant de filtre bactérien.

Comme illustré sur la figure 1, le tube de connexion 8 de sortie du sang filtré peut être relié à une tubulure externe de siphonage 9, aboutissant à un couvercle 10, dans lequel est également intégré le tube de connexion 7 pour l'entrée du sang à traiter et de l'air d'aspiration. Ce couvercle 10 est destiné à coopérer en position de service et de manière hermétique avec un appareil de récupération et de filtration du sang (non montré), par exemple du type de celui décrit dans le brevet EP 0.525.493 de la même titulaire.

D'autre part, en ce qui concerne la filtration proprement dite, la paroi 2 en PVC présentant les ouvertures précitées 4,5,6, est munie sur sa face interne d'un filtre- grille 11, de préférence en polyester, avec des mailles de l'ordre de 20 à 500 µm, par exemple d'environ 150 µm. Ce filtre-grille 11 est généralement traité à l'héparine et rendu hydrofuge pour limiter le processus de coagulation du sang.

La surface du filtre 11 est inférieure à celle de la paroi 2 sur laquelle il est fixé. Etant donné que le polyester formant ce filtre 11, de même que le polypropylène constituant le filtre bactérien dont est munie la sortie de l'air 5, ne sont pas appropriés pour une soudure directe simple sur du PVC, ces deux éléments filtres sont soudés à la paroi en PVC au moyen d'une contrepièce 12 également en PVC, les éléments-filtres étant ainsi pris en sandwich entre deux couches de PVC, la soudure étant réalisée de manière usuelle avec des machines de soudure à haute fréquence.

La contrepièce 12 présente une ouverture destinée à correspondre à l'ouverture de sortie 5 de l'air d'aspiration, ainsi que plusieurs ouvertures 13 allongées, horizontales, destinées au passage du sang filtré. Plus particulièrement, la contrepièce 12 en PVC est soudée sur la paroi 2 également en PVC, avec la grille-filtre 11 en sandwich, selon une soudure périphérique 14, une soudure sensiblement circulaire 15 autour de l'ouverture de sortie de l'air 5, et des îlots de soudure horizontaux 16 (voir figure 2), ces derniers permettant de maintenir le filtre 11 à proximité de la paroi 2.

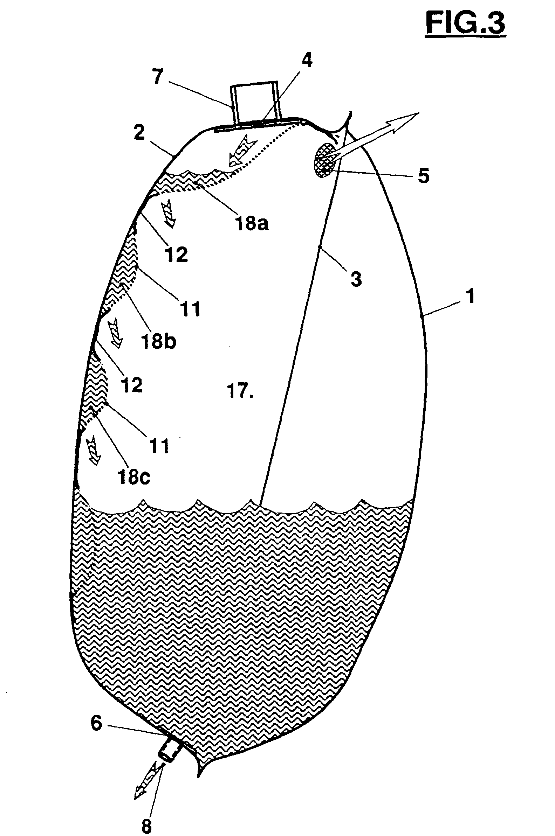

La disposition particulière de la grille filtre 11 par rapport à la paroi 2 a pour effet de créer, à l'intérieur de la poche souple de filtration, une première chambre 17 d'un volume proportionnellement important et destiné à recevoir le sang filtré, ainsi qu'une seconde chambre, d'un volume total faible par rapport à celui de la première chambre 17, et qui est en fait formée de plusieurs compartiments 18a, 18b, 18c, disposés horizontalement et communiquant les uns avec les autres, comme représenté schématiquement sur la figure 3. L'ouverture d'entrée du sang 4 débouche directement dans le compartiment supérieur 18a de la seconde chambre pour recevoir le sang à traiter.

Toujours en référence au schéma de la figure 3, le déroulement du processus de filtration est le suivant. La poche souple de filtration jetable doit bien entendu être tout d'abord introduite dans un appareil adéquat (non montré) et reliée à un dispositif d'aspiration et de régulation du vide. Le sang souillé (contenant par exemple des caillots, des débris d'os, etc) à filtrer est donc introduit par aspiration dans le compartiment supérieure 18a de la seconde chambre, par l'intermédiaire de l'ouverture d'entrée 4, puis toujours sous l'effet de l'aspiration par la mise "sous vide" de l'appareil, le sang passe à travers le filtre-grille 11 jusque dans la première chambre 17, depuis laquelle il peut être pompé par l'intermédiaire de l'ouverture de sortie 6.

Selon une variante (non illustrée), la chambre de rétention formée de plusieurs compartiments communicants 18a,18b,18c peut avantageusement être remplie partiellement ou complètement par des granules en matière plastique (PVC), par exemple d'environ 3 mm de diamètre, afin de réduire encore le volume de rétention, tout en produisant un effet de préfiltration. Une telle préfiltration peut s'avérer particulièrement utile pour défibrer le sang, avant que celui-ci ne passe à travers la grille filtrante 11 par exemple de 150 µm.

Ainsi, par rapport aux poches souples de filtration connues, par exemple du type de celle décrite dans le brevet EP 0.525.493, la poche selon la présente invention permet de remédier aux inconvénients de celles-ci :
- la disposition de la grille-filtre à proximité de la paroi permet de limiter le volume de rétention du sang;
- de ce fait, si le filtre commence à s'obstruer, la pression différentielle de part et d'autre augmente très rapidement, et le filtre peut alors se déboucher, et le fonctionnement de l'appareil est plus fiable;
- la répartition des îlots de soudure permet de supporter au maximum la pression d'éclatement lorsque le filtre est partiellement bouché;
- du point de vue de la fabrication, celle-ci est nettement facilitée du fait que l'ensemble des ouvertures et éléments additionnels (filtre notamment) sont prévus sur une seule des deux parois constitutives;
- enfin, la tubulure externe de siphonage permet d'éviter certains inconvénients d'une tubulure interne.

## Revendications

1. Poche souple jetable pour la récupération et la filtration du sang comportant deux chambres internes (17,18) séparées l'une de l'autre par une grille filtrante (11), une première chambre (17) étant reliée à l'extérieur par une ouverture de sortie (6) du sang filtré, et une seconde chambre (18) étant reliée à l'extérieur par une ouverture d'entrée (4) du sang à traiter, cette poche étant formée par deux parois (1,2) en matière plastique souple superposées et fixées par soudure de leurs rebords périphériques respectifs (3), **caractérisée par le fait que** les ouvertures d'entrée (4) et de sortie (6) du sang, ainsi qu'une ouverture de sortie (5) d'air d'aspiration munie d'un filtre bactérien, sont toutes pratiquées dans la même paroi (2) constitutive de la poche, et **par le fait que** la grille-filtre (11) ayant une surface inférieure à la surface de cette même paroi (2) coopère avec la face interne de celle-ci pour former plusieurs compartiments (18a, 18b, 18c) communiquant les uns avec les autres et constituant ladite seconde chambre, de telle sorte que le volume de rétention soit réduit.

2. Poche souple selon la revendication 1, **caractérisée par le fait que** la grille-filtre (11) est fixée contre la face interne de ladite paroi (2) au moyen d'une contre-pièce (12) en matière plastique présentant des ouvertures allongées (13) sensiblement horizontales.

3. Poche souple selon la revendication 2, **caractérisée par le fait que** la grille-filtre (11) est fixée à ladite paroi (2) par une soudure périphérique (14) entre cette paroi et la contre-pièce (12), ainsi que par des îlots de soudure (16) sensiblement horizontaux et délimitant les compartiments communiquants (18a, 18b, 18c).

4. Poche souple selon la revendication 3, **caractérisée par le fait que** le filtre bactérien est fixé sur l'ouverture de sortie (5) d'air d'aspiration également par une soudure périphérique (15) avec une ouverture pratiquée dans la contre-pièce (12).

5. Poche souple selon l'une des revendications 1 à 4, **caractérisée par le fait que** les compartiments communiquants (18a,18b,18c) formant ladite seconde chambre sont au moins partiellement remplis par des granules en matière plastique.

6. Poche selon l'une des revendications 1 à 5, **caractérisée par le fait qu'**elle comporte une tubulure externe (9) de siphonage reliant l'ouverture de sortie (6) du sang à la partie supérieure de la poche.

## Claims

1. Throwable blood bag for the recuperation and filtration of blood comprising two internal chambers (17, 18) separated one from the other by a filtering grid (11), a first chamber (17) being connected to the exterior by an exit opening (6) of filtered blood, and a second chamber (18) being connected to the exterior by an entrance opening (4) of blood to be treated, this bag being formed by two superposed walls (1, 2) made of flexible plastic material fixed at their respective peripheral edges (3) by welding, **characterised by** the fact that the entrance (4) and exit openings (6) as well as a exit opening (5) of aspiration air equipped with a bacteriological filter are all formed in the same constituting wall (2) of the bag, and by the fact that the filtering grid (11) having a surface smaller than the surface of this same wall (2) co-operates with the internal side of this one in order to form several compartments (18a, 18b, 18c) communicating the ones with the others and constituting said second chamber, in such a way that the retention volume is reduced.

2. Blood bag according to claim 1, **characterised by** the fact that the filtering grid (11) is fixed against the internal side of said wall (2) by means of a counter piece (12) made of plastic material presenting appreciably horizontal elongated openings (13).

3. Blood bag according to claim 2, **characterised by** the fact that the filtering grid (11) is fixed to said wall (2) by a peripheral welding (14) between this wall and the counter piece (12) as well as by small islands of welding (16) being appreciably horizontal and delimiting the communicating compartments (18a, 18b, 18c).

4. Blood bag according to claim 3, **characterised by** the fact that the bacteriological filter is fixed on the exit opening (5) of aspiration air as well by a peripheral welding (15) with an opening being formed in the counter piece (12).

5. Blood bag according to one of the claims 1 to 4, **characterised by** the fact that the communicating compartments (18a, 18b, 18c) forming said second chamber are at least partially filled with granules made of plastic material.

6. Blood bag according to one of the claims 1 to 5, **characterised by** the fact that it comprises an external siphoning tube (9) connecting the exit opening (6) of blood with the upper part of the bag.

## Patentansprüche

1. Wegwerfbarer Blutbeutel zur Wiedergewinnung und zum Filtern von Blut, der zwei interne Kammern (17, 18) aufweist, welche durch ein filterndes Gitter (11) voneinander separiert sind, wobei eine erste Kammer (17) durch eine Ausgangsöffnung (6) für das gefilterte Blut mit dem Äußeren verbunden ist und eine zweite Kammer (18) durch eine Eingangsöffnung (4) für das zu behandelnde Blut mit dem Äußeren verbunden ist, wobei dieser Beutel aus zwei übereinandergelegten und an ihren jeweiligen seitlichen Rändern (3) durch Verschweißen befestigten Wänden (1, 2) aus biegsamen Plastikmaterial gebildet ist, **dadurch gekennzeichnet, daß** die Eingangsöffnung (4) und die Ausgangsöffnung (6) für das Blut sowie eine mit einem Bakterienfilter ausgerüstete Ausgangsöffnung (5) für Saugluft alle in derselben konstituierenden Wand (2) des Beutels ausgeführt sind, und dadurch, daß das Filtergitter (11), das eine Oberfläche kleiner als die Oberfläche dieser gleichen Wand (2) hat, mit deren Innenseite zusammenspielt, um mehrere Maschen (18a, 18b, 18c) zu bilden, welche miteinander kommunizieren und besagte zweite Kammer bilden, derart, daß das Rückhaltevolumen reduziert wird.

2. Blutbeutel gemäß Anspruch 1, **dadurch gekennzeichnet, daß** das Filtergitter (11) gegen die Innenseite der besagten Wand (2) mittels eines Gegenstücks (12) aus Plastikmaterial, das merklich horizontale, längliche Öffnungen (13) aufweist, befestigt ist.

3. Blutbeutel gemäß Anspruch 2, **dadurch gekennzeichnet, daß** das Filtergitter (11) durch eine Schweißnaht an seinem Rand (14) zwischen dieser Wand und dem Gegenstück (12) sowie durch kleine Inselchen von Schweißnähten (16), die merklich horizontal sind und die kommunizierenden Maschen (18a, 18b, 18c) abgrenzen, an die besagte Wand (2) befestigt ist.

4. Blutbeutel gemäß Anspruch 3, **dadurch gekennzeichnet, daß** der Bakterienfilter ebenso durch eine Schweißnaht an seinem Rand (15) auf der Ausgangsöffnung (5) für Saugluft befestigt ist, wobei eine Öffnung im Gegenstück (12) ausgebildet ist.

5. Blutbeutel gemäß einem der Ansprüche 1 bis 4, **dadurch gekennzeichnet, daß** die kommunizierenden Maschen (18a, 18b, 18c), welche die besagte zweite Kammer bilden, zumindest teilweise mit Körnchen aus Plastikmaterial angefüllt sind.

6. Blutbeutel gemäß einem der Ansprüche 1 bis 5, **dadurch gekennzeichnet, daß** er eine externe Saugleitung (9) aufweist, welche die Ausgangsöffnung (6) für das Blut mit dem oberen Teil des Beutels verbindet.
